(19) Europäisches Patentamt

European Patent Office

Office européen des brevets

(11) **EP 3 162 344 B1**

(12) **EUROPEAN PATENT SPECIFICATION**

(45) Date of publication and mention
of the grant of the patent:
**06.11.2019 Bulletin 2019/45**

(21) Application number: **15816050.7**

(22) Date of filing: **10.04.2015**

(51) Int Cl.:
***A61F 13/53*** *(2006.01)*

(86) International application number:
**PCT/JP2015/061268**

(87) International publication number:
**WO 2016/002299 (07.01.2016 Gazette 2016/01)**

(54) **ABSORBENT BODY FOR BODY FLUID-ABSORBING ARTICLES**

ABSORBIERENDER KÖRPER FÜR KÖRPERFLÜSSIGKEITSABSORBIERENDE ARTIKEL

CORPS ABSORBANT POUR ARTICLES D'ABSORPTION DE LIQUIDE ORGANIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.06.2014 JP 2014134209**

(43) Date of publication of application:
**03.05.2017 Bulletin 2017/18**

(73) Proprietor: **Unicharm Corporation
Ehime 799-0111 (JP)**

(72) Inventors:
• **UDA, Masashi
Kanonji-shi
Kagawa 769-1602 (JP)**

• **MARUYAMA, Takashi
Kanonji-shi
Kagawa 769-1602 (JP)**

(74) Representative: **Dolleymores
9 Rickmansworth Road
Watford, Hertfordshire WD18 0JU (GB)**

(56) References cited:
**JP-A- H08 269 869          JP-A- 2014 079 561
JP-A- 2014 113 191          US-A- 4 590 114
US-A- 5 458 835            US-A1- 2001 051 485
US-A1- 2003 236 511        US-A1- 2006 134 386**

## Description

TECHNICAL FIELD

The present invention relates to an absorbent body for a body liquid-absorbing article. More specifically, it relates to an absorbent body to be used in a body liquid-absorbing article such as a sanitary napkin.

BACKGROUND ART

Absorbent bodies for body liquid-absorbing articles are known, which have an absorbent retaining layer comprising fluff pulp, a super-absorbent polymer and heat sealable synthetic resin fibers, and a nonwoven fabric layer composed of heat sealable synthetic resin fibers which is situated on the front sheet side of the absorbent retaining layer (JP 2002-11047 A). In the absorbent body described in JP 2002-11047 A, the heat sealable synthetic resin fibers in the absorbent retaining layer are tangled or heat-fused together, and the heat sealable synthetic resin fibers in the absorbent retaining layer and the heat sealable synthetic resin fibers in the nonwoven fabric layer are heat-fused, in order to prevent deformation of the absorbent body during use of the absorbent article.

PRIOR ART DOCUMENTS

PATENT DOCUMENTS

Patent Document 1: JP 2002-11047 A
US 2003/236511 A1 discloses a process for forming a densified nonwoven web. It comprises the steps of forming an open structure comprising at least about 5 wt% of cellulosic materials; heating the open structure to a temperature of at least about 40° C while substantially maintaining moisture present in the open structure; compressing at least a portion of the heated open structure to form the densified nonwoven web having portions with a local density of greater than about 0.2 g/cm$^3$; and releasing the densified nonwoven web from compression.

SUMMARY OF THE INVENTION

PROBLEMS TO BE SOLVED BY THE INVENTION

In JP 2002-11047 A, to prevent disintegration of the body fluid-absorbent retaining layer, the design is such as to impart anti-deformation strength to the absorbent body, by heat-fusion of the absorbent retaining layer and the nonwoven fabric layer at their contact surfaces, thereby improving the bonding strength between them, and heat fusion of the fusible synthetic resin fibers together in the absorbent retaining layer. With the absorbent body described in JP 2002-11047 A, however, the degree of heat fusion is increased thereby strengthening the absorbent body but also tending to render the absorbent body hard.
It is an object of the present invention to provide an absorbent body for a body liquid-absorbing article, that exhibits both softness and strength.

MEANS FOR SOLVING THE PROBLEMS

The present inventors have found that by arranging non-fused compressed sections in a repeating pattern on an absorbent body, it is possible to obtain an absorbent body exhibiting both softness and strength, and the invention has thus been completed.
The present invention provides the absorbent body of independent claim 1 and the body liquid-absorbing article of independent claim 7. The dependent claims specify preferred but optional features.
In other words, the invention is an absorbent body for a body liquid-absorbing article with an apparent density of 0.06 to 0.14 g/cm$^3$, comprising 100 parts by weight of cellulose-based water-absorbent fibers and 7.5 to 100 parts by weight of thermoplastic resin fibers, wherein the thermoplastic resin fibers having longer fiber lengths than the cellulose-based water-absorbent fibers are mutually entangled, and the absorbent body has compressed sections arranged in a repeating pattern, the compressed sections being non-fused and the absorbent body having a Gurley bending resistance of no greater than 1 mN, the area of each individual compressed section is 0.1 to 20.0 mm$^2$, and the proportion of the area of the compressed sections with respect to the area of the absorbent body is 1 to 20%
The invention encompasses the following aspects.

[1] An absorbent body for a body liquid-absorbing article with an apparent density of 0.06 to 0.14 g/cm$^3$, comprising

100 parts by weight of cellulose-based water-absorbent fibers and 7.5 to 100 parts by weight of thermoplastic resin fibers, wherein the thermoplastic resin fibers having longer fiber lengths than the cellulose-based water-absorbent fibers are mutually entangled, and the absorbent body has compressed sections arranged in a repeating pattern, the compressed sections being non-fused and the absorbent body having a Gurley bending resistance of no greater than 1 mN, the area of each individual compressed section is 0.1 to 20.0 mm$^2$, and the proportion of the area of the compressed sections with respect to the area of the absorbent body is 1 to 20%

[2] An absorbent body according to [1], wherein the tensile strength of the absorbent body is 0.4 N/cm or greater.

[3] An absorbent body according to [1] or [2], wherein the displacement at maximum load of the absorbent body is 10 to 23 mm.

[4] An absorbent body according to any one of [1] to [3], wherein the absorbent body further includes a super-absorbent polymer.

[5] An absorbent body according to any one of [1] to [4], wherein the basis weight of the absorbent body is 40 to 900 g/m$^2$.

[6] An absorbent body according to any one of [1] to [5], wherein at least some of the thermoplastic resin fibers have a first section that is exposed on one side of the absorbent body, a second section that is exposed on the other side of the absorbent body, and a connecting section that connects the first section and the second section.

[7] A body liquid-absorbing article comprising an absorbent body according to any one of [1] to [6].

EFFECT OF THE INVENTION

The absorbent body of the invention exhibits both softness and strength. An absorbent body exhibiting the conflicting properties of softness and strength reduces the discomfort that a wearer feels when wearing an absorbent article, and because it has sufficient strength, the absorbent body does not twist or tear during use.

BRIEF DESCRIPTION OF THE DRAWINGS

Fig. 1 is a plan view showing an example of an absorbent body of the invention.
Fig. 2 is a cross-sectional schematic diagram along line I-I' of Fig. 1.
Fig. 3 is a diagram showing an example of a production apparatus for an absorbent body of the invention.
Fig. 4 is a diagram illustrating a method of measuring tensile strength in the thickness direction of the absorbent body.

MODE FOR CARRYING OUT THE INVENTION

The invention will now be described with reference to the accompanying drawings, with the understanding that the invention is not limited to the examples depicted in the drawings.

The invention relates to an absorbent body for a body liquid-absorbing article with an apparent density of 0.06 to 0.14 g/cm$^3$, comprising 100 parts by weight of cellulose-based water-absorbent fibers and 7.5 to 100 parts by weight of thermoplastic resin fibers, wherein the thermoplastic resin fibers having longer fiber lengths than the cellulose-based water-absorbent fibers are mutually entangled, and the absorbent body has compressed sections arranged in a repeating pattern, the compressed sections being non-fused and the absorbent body having a Gurley bending resistance of no greater than 1 mN.

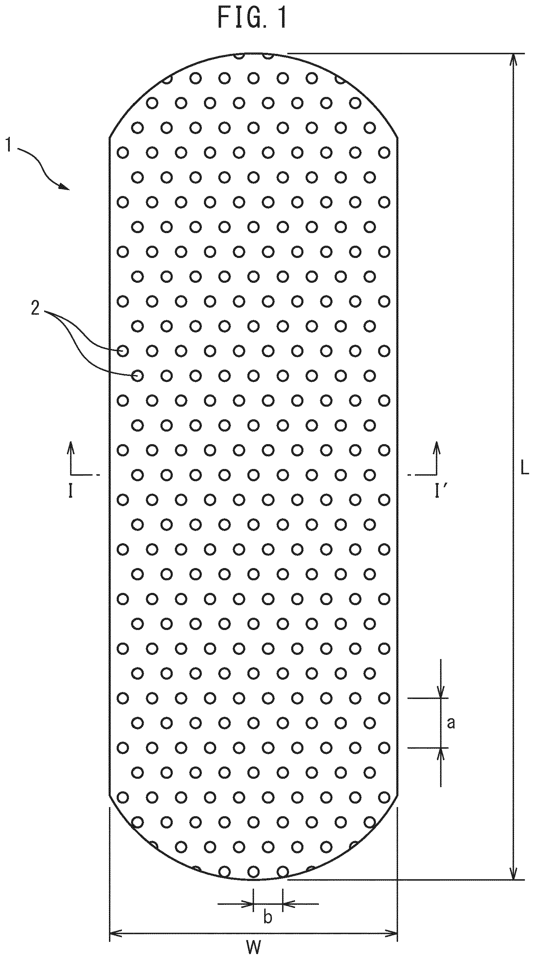

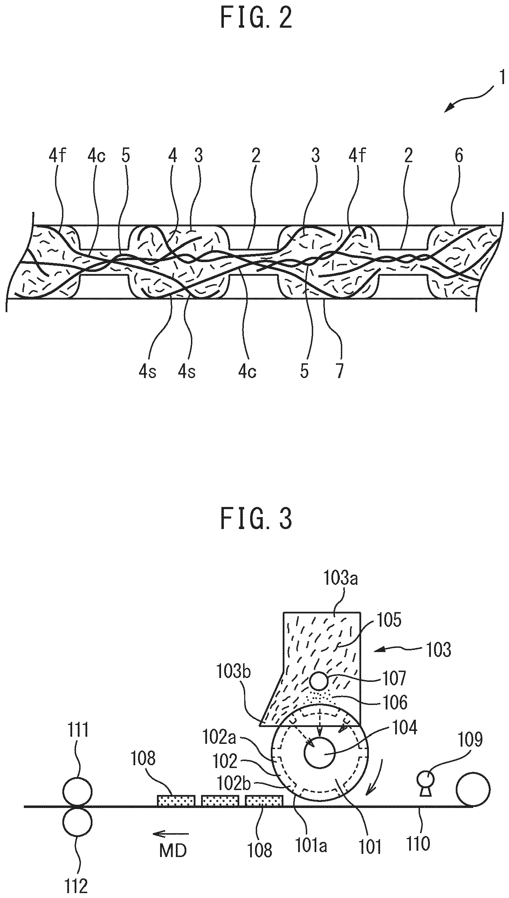

Fig. 1 is a plan view showing an example of an absorbent body of the invention, and Fig. 2 is a cross-sectional schematic diagram along line I-I' of Fig. 1. The absorbent body 1 has compressed sections 2 arranged in a repeating pattern. The compressed sections are sections with high apparent density, formed by pressurized compaction. The compressed sections can be formed by embossing at a temperature below the melt start temperature of the thermoplastic resin fibers. The compressed sections are non-fused. The absorbent body 1 includes cellulose-based water-absorbent fibers 3 and thermoplastic resin fibers 4. The thermoplastic resin fibers 4 have longer fiber lengths than the cellulose-based water-absorbent fibers 3. The thermoplastic resin fibers 4 are also mutually entangled. Here, "entangled" means that the fibers are tangled together. In Fig. 2, the thermoplastic resin fibers 4 have entangled sections 5 in which the thermoplastic resin fibers are mutually entangled. The thermoplastic resin fibers 4 are mutually entangled but are not fused. The thermoplastic resin fibers 4 are also non-fused at the compressed sections. For explanation in Fig. 2, the thermoplastic resin fibers 4 are illustrated as being thick and long and the cellulose-based water-absorbent fibers 3 are illustrated as being thin and short, but this does not reflect the actual thicknesses and lengths of the fibers.

Since the absorbent body of the invention has an apparent density of 0.06 to 0.14 g/cm$^3$ and is not very compacted, softness is imparted by the non-collapsed pulp, while softness is also provided by the long fiber lengths of the thermoplastic resin fibers and the fact that the thermoplastic resin fibers are not fused, such that the bending resistance is 1 mN or lower. On the other hand, since the thermoplastic resin fibers are mutually entangled and have compressed sections, the strength necessary for an absorbent body for a body liquid-absorbing article can be exhibited, while it is also resistant to deformation and does not twist or tear. In other words, since the absorbent body of the invention has increased strength of the absorbent body without being dependent on heat fusion of the thermoplastic resin fibers, it is softer than absorbent bodies of the prior art that prevent twisting of the absorbent bodies by heat fusion of the thermoplastic resin fibers, and thus the person wearing the absorbent article is less likely to experience discomfort.

The arrangement pattern of the compressed sections is not restricted so long as it is a repeating pattern, and examples of such patterns include 60° zigzag arrangements (as on the 3 side of a die), square zigzag arrangements (as on the 5 side of a die), and parallel arrangements (as on the 4 or 6 side of a die). The arrangement of compressed sections shown in Fig. 1 is an example of a 60° zigzag arrangement.

The shapes of the compressed sections are not particularly restricted, and examples include circular, elliptical, square, rectangular, rhomboid, triangular, star and heart shapes, among which circular shapes are preferred. Incidentally, it is not essential for all of the compressed sections to be of the same shape, and compressed sections with different shapes may be combined.

The proportion of the area of the compressed sections with respect to the area of the absorbent body (hereunder also referred to as "compressed section area ratio") is 1 to 20%, preferably 2 to 15% and more preferably 3 to 10%. If the compressed section area ratio is less than 1% the effect of the compressed sections will tend to be less apparent, and if the compressed section area ratio is greater than 20% the wearer will tend to feel the hardness of the absorbent body. The terms "area of the absorbent body" and "area of the compressed sections" refer to the area when viewing the plane of the absorbent body.

The area of each individual compressed section is 0.1 to 20.0 mm$^2$, preferably 1.0 to 15.0 mm$^2$ and more preferably 2.0 to 10.0 mm$^2$. If the area of each individual compressed section is less than 0.1 mm$^2$ the protrusions of the embossing roll will be acute angles and the absorbent body may be torn, while if the area of the individual compressed sections is greater than 20.0 mm$^2$ the absorbent body will tend to become hard.

The center distance between the two closest adjacent compressed sections is preferably 4 to 20 mm, more preferably 5 to 15 mm and even more preferably 6 to 12 mm. If the center distance is too short, the absorbent body will increase in density and become hard, tending to create discomfort for the wearer. If the center distance is too long, it may no longer be possible to impart adequate strength to the absorbent body.

The thickness of the compressed sections is preferably 0.01 to 50%, more preferably 0.1 to 25% and even more preferably 0.5 to 10% of the thickness of the absorbent body at the sections other than the compressed sections. If the thickness of the compressed sections is too low, the absorbent body may be hard creating discomfort for the wearer, and if the thickness of the compressed sections is too high, the strength may be deficient and the absorbent body may undergo twisting and tearing.

The absorbent body of the invention is an absorbent body for a body liquid-absorbing article. Here, the term "body liquid-absorbing article" refers to an absorbent article for absorption of body fluids such as menstrual blood or urine, and more specifically there may be mentioned sanitary napkins and urine-absorbing pads.

Cellulose-based water-absorbent fibers may be pulp, such as wood pulp obtained using a conifer or broadleaf tree starting material, or nonwood pulp such as bagasse, kenaf, bamboo, hemp, cotton (for example, cotton linter); regenerated cellulose fiber such as rayon fiber, or semisynthetic fiber such as acetate fiber. The pulp is preferably Kraft pulp which is industrially economical and highly safe. Particularly preferred is ground pulp with a fiber length of about 3 mm.

The thermoplastic resin fibers may be ones containing only a single component, such as simple fibers, or ones containing multiple components such as composite fibers. Such components include polyolefins such as polyethylene (PE), polypropylene (PP) and polybutylene, ethylene-vinyl acetate copolymer, ethylene-ethyl acrylate copolymer, ethylene-acrylic acid copolymer and ionomer resins; polyesters such as polyethylene terephthalate (PET), polybutylene terephthalate (PBT), polytrimethylene terephthalate (PTT) and polylactic acid; and polyamides such as nylon 6 and nylon 66.

Examples of composite fibers include composite fibers such as core-sheath fibers, side-by-side fibers and sea/island fibers, and hollow type fibers; irregularly shaped fibers such as flat fibers, Y-shaped fibers or C-shaped fibers; solid crimped fibers such as latent crimped or developed crimped fibers, and split fibers that have been split by a physical load such as a water stream, heat, embossing or the like, preferred among which are core-sheath fibers and especially PET/PE and PP/PE (core/sheath), which are industrially economical and highly safe. The weight ratio of the core component/sheath component is preferably 10/90 to 90/10 and more preferably 30/70 to 70/30. If the sheath component proportion is low the fusibility will be reduced, and if the sheath component proportion increases, the spinnability will tend to be reduced.

The absorbent body includes 100 parts by weight of cellulose-based water-absorbent fibers and 7.5 to 100 parts by weight of thermoplastic resin fibers. That is, the content of the thermoplastic resin fibers in the absorbent body is 7.5 to 100 parts by weight, preferably 10 to 100 parts by weight and more preferably 40 to 100 parts by weight, with respect to 100 parts by weight of the cellulose-based water-absorbent fibers. If the content of the thermoplastic resin fibers is too low, it may no longer be possible to impart adequate strength to the absorbent body. If the content of the thermoplastic resin fibers is too high, the liquid absorption property of the absorbent body will tend to be insufficient.

The mean fiber length of the thermoplastic resin fibers is longer than the mean fiber length of the cellulose-based water-absorbent fibers.

The mean fiber length of the thermoplastic resin fibers is preferably longer than half of the center distance between the two closest adjacent compressed sections, more preferably it is longer than the center distance between the two closest adjacent compressed sections, and even more preferably it is longer than twice the center distance between the two closest adjacent compressed sections. If the mean fiber length of the thermoplastic resin fibers is shorter than half the center distance between the two closest adjacent compressed sections, thermoplastic resin fibers anchored to different compressed sections will not become entangled and it will be difficult to increase the strength of the absorbent body. If the mean fiber length of the thermoplastic resin fibers is longer than twice the center distance between the two closest adjacent compressed sections, one thermoplastic resin fiber can become anchored to multiple compressed sections, thereby more easily increasing the strength of the absorbent body. The absolute value of the mean fiber length of the thermoplastic resin fibers is not restricted so long as it is longer than 1/2 of the center distance between the two closest adjacent compressed sections, but it is preferably 8 to 50 mm, more preferably 10 to 40 mm and even more preferably 12 to 30 mm. If the mean fiber lengths of the thermoplastic resin fibers are too long, the fiber-opening property of the thermoplastic resin fibers will be notably reduced and the absorbent body will include non-opened thermoplastic resin fibers, thus tending to lower the uniformity of the absorbent body.

The mean fiber length of the cellulose-based water-absorbent fibers is preferably shorter than the center distance between the two closest adjacent compressed sections. If the mean fiber length of the cellulose-based water-absorbent fibers is longer than the center distance between the two closest adjacent compressed sections, it may become difficult to obtain softness for the absorbent body. The absolute value of the mean fiber length of the cellulose-based water-absorbent fibers is not restricted so long as it is shorter than the center distance between the two closest adjacent compressed sections, but it is preferably 0.5 to 7 mm, more preferably 1 to 6 mm and even more preferably 2 to 5 mm. If the mean fiber length of the cellulose-based water-absorbent fibers is too short, the absorption property may be poor and a feeling of discomfort may be created for the wearer.

The mean fiber length for non-pulp thermoplastic resin fibers and cellulose-based water-absorbent fibers, such as regenerated cellulose fibers or semisynthetic fibers, is measured according to JIS L 1015:2010, Appendix A, "A7.1 Measurement of Fiber Length", "A7.1.1 Method A (standard method) Method measuring individual fiber lengths on scaled glass plate". This method, incidentally, is the test method corresponding to ISO 6989 published in 1981.

The mean fiber length of pulp is the weight-weighted average fiber length, and it is the L(w) value measured using Kajaani fiber Lab fiber properties (off-line)] by Metso Automation.

The size of the thermoplastic resin fibers is not restricted but is preferably 0.5 to 10 dtex and more preferably 1.5 to 5 dtex. If the size is too small, the fiber-opening property of the thermoplastic resin fibers may be reduced, and if the size is too large there will be fewer thermoplastic resin fibers, tending to result in fewer points of entanglement with other thermoplastic resin fibers.

By mixing thermoplastic resin fibers with fiber lengths of 8 to 50 mm with cellulose-based water-absorbent fibers in an absorbent body composed mainly of cellulose-based water-absorbent fibers (for example, ground pulp with a fiber length of about 3 mm), it is possible to increase the displacement at maximum load and form an entangled structure of the mixed thermoplastic resin fibers. Forming an entangled structure improves the strength. In addition, since the fibers are not heat-fused together it is possible to lower the Gurley bending resistance.

The thickness of the absorbent body at the sections other than the compressed sections is preferably 0.1 to 15 mm, more preferably 1 to 10 mm and even more preferably 2 to 5 mm.

The thickness (mm) of the absorbent body before creation of the compressed sections can be measured using an FS-60DS thickness gauge by Daiei Kagaku Seiki Mfg. Co., Ltd. (measuring surface: 44 mm-diameter circular, measuring pressure: 3 g/cm$^2$), with five different locations of the sample being pressed at a pressure of 3 g/cm$^2$ under standard conditions (temperature: 23±2°C, relative humidity: 50±5%), the thickness being measured after 10 seconds of pressing, and the mean value of the five measured values being recorded as the thickness of the absorbent body.

The thickness (mm) of the absorbent body having the compressed sections can be measured by microscope observation of the cross-section parallel to the direction of thickness direction of the absorbent body.

The basis weight of the absorbent body is preferably 40 to 900 g/m$^2$, more preferably 50 to 800 g/m$^2$ and even more preferably 100 to 500 g/m$^2$. This is from the viewpoint of the strength and absorption property of the absorbent body.

The basis weight is measured according to JIS L 1913:2010 "6.2 Weight per unit area (ISO method)".

The apparent density of the absorbent body is preferably 0.06 to 0.14 g/cm$^3$, more preferably 0.07 to 0.12 g/cm$^3$ and even more preferably 0.08 to 0.1 g/cm$^3$. If the absorbent body has the proportions of cellulose-based water-absorbent fibers and thermoplastic resin fibers specified above and the apparent density specified above, the liquid absorption property of the absorbent body will tend to be excellent.

The apparent density of the absorbent body can be calculated from the basis weight and thickness of the absorbent body.

In the absorbent body of the invention, preferably at least some of the thermoplastic resin fibers have a first section that is exposed on one side of the absorbent body, a second section that is exposed on the other side of the absorbent body, and a connecting section that connects the first section and the second section.

In Fig. 2, the thermoplastic resin fibers 4 have a first section 4f that is exposed on one side 6 of the absorbent body, a second section 4s that is exposed on the other side 7 of the absorbent body, and a connecting section 4c that connects the first section 4f and the second section 4s.

The term "exposed" used in relation to the thermoplastic resin fibers means that the thermoplastic resin fibers are present on at least the surface of the absorbent body.

At least some of the thermoplastic resin fibers have a first section exposed on one side of the absorbent body, a second section exposed on the other side of the absorbent body and a connecting section connecting the first section and the second section, whereby the thermoplastic resin fibers function as a lattice to hold the other components of the absorbent body such as cellulose-based water-absorbent fibers, thereby improving the strength of the absorbent body. As a result, when the force of body pressure or the like is applied, there is less detachment inside the absorbent body, and the absorbent body becomes more resistant to twisting than an absorbent body containing no thermoplastic resin fibers, such as an absorbent body containing pulp alone.

Furthermore, since at least some of the thermoplastic resin fibers have a first section exposed on one side of the absorbent body, a second section exposed on the other side of the absorbent body and a connecting section connecting the first section and the second section, when the absorbent body in the absorbent article comprising an absorbent body of the invention is bonded to the layer that is to be adjacent to the wearer side of the absorbent body (for example, a liquid-permeable layer) and/or the layer that is to be adjacent to the clothing side of the absorbent body (for example, a liquid-impermeable layer), this helps prevent interlayer detachment inside the absorbent body and thus renders the absorbent body resistant to twisting.

The absorbent body may also include a super-absorbent polymer. A super-absorbent polymer, also known as SAP, has a three-dimensional network structure with an appropriately crosslinked water-soluble polymer and therefore absorbs a few hundred to a few thousand times its weight of water, but it is essentially water-insoluble and the absorbed water does not emerge even with a certain degree of pressure application; commercially available types thereof include starch-based, acrylic acid-based and amino acid-based super-absorbent polymers. The content of the super-absorbent polymer and/or high water-absorbent fibers is not particularly restricted, but it is preferably 5 to 150 parts by weight, more preferably 10 to 100 parts by weight and even more preferably 20 to 60 parts by weight with respect to 100 parts by weight of the cellulose-based water-absorbent fibers.

The tensile strength of the absorbent body is preferably 0.4 N/cm or greater and more preferably 0.6 to 0.9 N/cm. If the tensile strength is too low, the absorbent body may undergo twisting or tearing during use. If the tensile strength is too high, the absorbent body may become hard creating discomfort for the wearer.

The displacement at maximum load of the absorbent body is preferably in the range of 10 to 23 mm. If the displacement at maximum load is too small, the absorbent body itself will lack ductility, and the absorbent body will readily tear when deformed.

The displacement at maximum load referred to here is the elongation at maximum load when measuring the tensile strength of the absorbent body.

The displacement at maximum load is a measure of the degree of entangling, with a greater displacement at maximum load corresponding to a higher degree of entangling.

The Gurley bending resistance of the absorbent body is preferably no greater than 1 mN. The Gurley bending resistance is a measure of the flexural strength, with a lower Gurley bending resistance corresponding to lower flexural strength and easier bending. There is no particular restriction on the lower limit of the Gurley bending resistance, but the Gurley bending resistance will usually be 0.2 mN or greater, with the more preferred range for the Gurley bending resistance being 0.4 to 1 mN.

The Gurley bending resistance is measured according to JIS L 1913 6.7.3.

The lower limit for the tensile strength in the thickness direction of the absorbent body is preferably 100 Pa or greater, more preferably 150 Pa or greater, even more preferably 200 Pa or greater and even more preferably 250 Pa or greater. If the tensile strength in the thickness direction of the absorbent body is lower than 100 Pa, the strength of the absorbent body will tend to be weak and the absorbent body will be more prone to twisting. The upper limit for the tensile strength in the thickness direction of the absorbent body is also not particularly restricted but is preferably no greater than 3000 Pa from the viewpoint of softness.

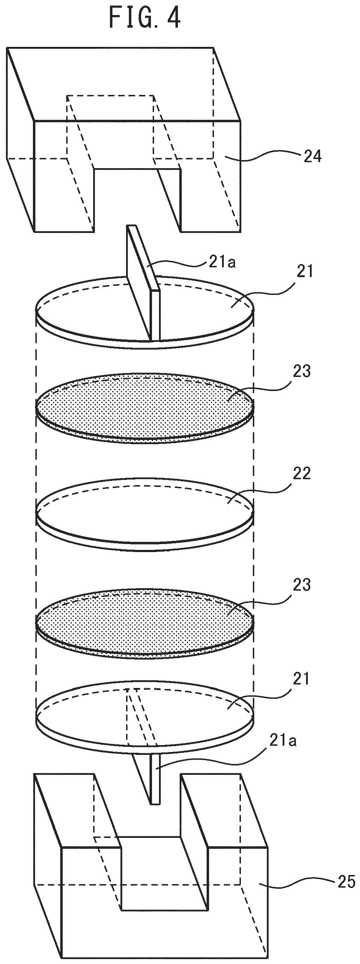

The tensile strength in the thickness direction of the absorbent body is measured as follows, using a device as illustrated in Fig. 4.

(1) A pair of acrylic resin jigs 21 (diameter: 68 mm, weight of each jig: 200 g, grip section 21a height: 50 mm) is prepared.

(2) A sample 22 with a diameter of 68 mm is prepared from the absorbent body.

(3) Two strips of double-sided tape 23 (3M Corp., adhesive transfer tape 950) cut out to a diameter of 68 mm are prepared.

(4) The sample 22 is affixed to the pair of jigs 21 using the two double-sided tape pieces 23, as shown in Fig. 4.

(5) The pair of jigs 21 with the sample 22 is placed on a holding stage 25, and a weight 24 (10.5 kg) is set over it and allowed to stand for 3 minutes.

(6) The pair of jigs 21 is set in a tensile tester (Shimadzu Corp., AG-1kNI) with a grip spacing of 70 mm.

(7) The sample 22 is subjected to a tensile test at a speed of 100 mm/min until the inner layers of the sample 22 detach, and the maximum tensile force (N) at that time is recorded.

(8) The measurement is repeated for a total of 5 times, the average value for the maximum tensile force (N) is determined, and the tensile strength (Pa) is calculated by the following formula.

$$\text{Tensile strength (Pa)} = \text{Average of maximum tensile force (N)}/0.003632 \ (m^2)$$

The measurement is conducted under 20°C conditions.

A production process for an absorbent body of the invention will now be explained.

Fig. 3 is a diagram showing an example of a production apparatus for an absorbent body of the invention.

Incidentally, the thermoplastic resin fibers and cellulose-based water-absorbent fibers are not shown separately in Fig. 3.

A plurality of recesses 102 are formed at a prescribed pitch on the peripheral surface 101a of a rotating suction drum 101, extending from the peripheral surface 101a toward the center of the suction drum, as a molding form in which the absorbent body material is to be packed. When the suction drum 101 is rotated and the recesses 102 approach the material feeder 103, the suction action of the suction section 104 causes the absorbent body material supplied from the material feeder 103 to accumulate in the recesses 102.

The material feeder 103, equipped with a hood 103a, is formed so as to cover the suction drum 101, and the material feeder 103 supplies a mixture 105 comprising cellulose-based water-absorbent fibers and thermoplastic resin fibers into the recesses 102 by air transport. The material feeder 103 is also provided with a super-absorbent polymer feeder 107 that supplies a super-absorbent polymer 106, so that the super-absorbent polymer 106 is supplied to the recesses 102. The mixture 105 of the cellulose-based water-absorbent fibers and thermoplastic resin fibers, and the super-absorbent polymer particles 106 (hereunder referred to as "absorbent body starting material") accumulates in the recesses 102 in a mixed state, and an absorbent body 108 is formed in the recesses 102. When an absorbent body not containing a super-absorbent polymer is to be produced, either supply of the super-absorbent polymer from the super-absorbent polymer feeder is halted, or an apparatus without a super-absorbent polymer feeder may be used.

The important aspect during production for mutually entangling the thermoplastic resin fibers is that the rotational speed of the suction drum is slower than the rate of spraying the absorbent body material, when the absorbent body material containing the thermoplastic resin fibers is accumulated in the recesses 102 of the suction drum 101. If the rate of spraying the absorbent body material is equal to the rotational speed of the suction drum, or if the rotational speed of the suction drum is faster, then the thermoplastic resin fibers will more easily be oriented and aligned in the machine direction MD, and be resistant to entangling.

In order for at least some of the thermoplastic resin fibers to have a first section exposed on one side of the absorbent body, a second section exposed on the other side of the absorbent body and a connecting section connecting the first section and the second section (hereunder also referred to as "specified orientation"), the hood 103a of the material feeder 103 has an accumulation space 103b that is larger than hoods generally used in the technical field, at the downstream end in the machine direction MD (upstream from accumulation of the absorbent body starting material). This will tend to cause the thermoplastic resin fibers to accumulate in the depthwise direction of the recesses 102, or in other words, in the direction from the peripheral surface 101a of the suction drum 101 toward the center. As regards the absorbent body starting material and the recesses 102, the absorbent body starting material tends to accumulate in order from the recess section 102a of the recess at the downstream end of rotation of the suction drum toward the recess section 102b at the upstream end of rotation, and therefore the thermoplastic resin fibers tend to have the aforementioned specified orientation.

In addition, during accumulation of the absorbent body starting material in the recesses 102, the thermoplastic resin fibers can be easily given the aforementioned specified orientation by increasing the suction force of the suction drum upstream from accumulation of the absorbent body starting material, so that it is greater than downstream from accumulation of the absorbent body starting material.

Furthermore, if the rotational speed of the suction drum is slower than the rate of flow of the absorbent body starting material, the thermoplastic resin fibers will more easily adopt the aforementioned specified orientation.

Next, the absorbent body 108 formed in the recesses 102 is transported onto a carrier sheet 110 having an adhesive coated by a coating machine 109.

The carrier sheet 110 later forms a nonwoven fabric layer between the absorbent body and the liquid-impermeable layer, but in an embodiment where the body liquid-absorbing article does not have a nonwoven fabric layer, the liquid-permeable layer, the liquid-impermeable layer or an optional auxiliary sheet or the like may be used as the carrier sheet.

Next, the absorbent body 108 placed on the carrier sheet 110 is embossed by a pair of embossing rolls 111, 112, forming compressed sections 2. The pair of embossing rolls may be composed of, for example, a roll with patterned heights, and a flat roll.

The embossing is accomplished at a temperature of no higher than the melt start temperature of the thermoplastic resin fibers, such as a room temperature. When the embossing is to be done at room temperature, the embossing roll is not heated. The tips of the heights of the embossing roll and the gaps in the flat roll are preferably 0 to 2 mm, more preferably 0.05 to 1 mm and even more preferably 0.1 to 0.5 mm. The processing time for embossing is preferably 0.0001 to 5 seconds and more preferably 0.005 to 2 seconds.

The present invention further relates to a body liquid-absorbing article comprising the absorbent body described above. As already explained, the term "body liquid-absorbing article" refers to an absorbent article for absorption of body fluids such as menstrual blood or urine, and more specifically there may be mentioned sanitary napkins and urine-absorbing pads. The structure of the body liquid-absorbing article of the invention is not restricted so long as it contains the absorbent body of the invention, and for example, it may be composed of the absorbent body of the invention and a liquid-permeable sheet and liquid-impermeable sheet sandwiching it.

In the absorbent article of the invention, the cellulose-based water-absorbent fibers contained in the absorbent body are associated with the fluid absorption and fluid retention of the absorbent body, while the thermoplastic resin fibers in the absorbent body are associated with connection between the liquid-permeable sheet and/or liquid-impermeable sheet, and especially the liquid-impermeable layer, and this prevents deformation of the absorbent article and imparts flexibility to the absorbent body and thus to the absorbent article.

The body liquid-absorbing article of the invention may be produced by an established method. For example, it may be produced by placing the absorbent body of the invention on a liquid-impermeable sheet and further layering a liquid-permeable sheet over it.

EXAMPLES

The following fibers were used in the examples. Pulp: NB416 by Warehouser, fiber length: about 2 to 3 mm Thermoplastic resin fibers: core-sheath fiber by JNC Corp., core: polyethylene terephthalate (PET), sheath: high-density polyethylene (HDPE), core-sheath ratio: 50:50, titanium oxide content: 0.7% in core, size: 2.2 dtex, number of crimps: 12 $\pm$2/inch, fiber length: 30 mm, 25 mm, 20 mm, 12 mm or 6 mm.

Example 1

Pulp fiber was opened and accumulated on a suction drum to form a pulp web with a basis weight of 200 g/m², while separately, thermoplastic resin fibers with fiber lengths of 30 mm are opened and accumulated on a suction drum, to form a thermoplastic resin fiber web having a basis weight of 40 g/m², the pulp web and the thermoplastic resin fiber web are layered, and then the layered web was opened and accumulated on an absorbent body-forming drum to obtain a molded article having the shape shown in Fig. 1 (L = 200 mm, W = 70 mm).

The thickness of the molded article was adjusted by flat pressing, and then subjected to embossing treatment through an embossing roll and plain roll, after which compressed sections (compressed section area ratio: 10.8%, a = 12 mm, b = 7 mm, tip diameter: 2.4 mm, room temperature) were molded in the pattern shown in Fig. 1, to fabricate an absorbent body.

Example 2

An absorbent body was fabricated in the same manner as Example 1, except that thermoplastic resin fibers with a fiber length of 25 mm were used instead of thermoplastic resin fibers with a fiber length of 30 mm.

Example 3

An absorbent body was fabricated in the same manner as Example 1, except that thermoplastic resin fibers with a fiber length of 20 mm were used instead of thermoplastic resin fibers with a fiber length of 30 mm.

Example 4

An absorbent body was fabricated in the same manner as Example 1, except that thermoplastic resin fibers with a fiber length of 12 mm were used instead of thermoplastic resin fibers with a fiber length of 30 mm.

Comparative Example 1

An absorbent body was fabricated in the same manner as Example 1, except that hot air treatment was carried out instead of embossing treatment. The hot air treatment was carried out by a through-air method, at a temperature of 135°C, an airflow rate of 5 m/sec and a heating time of 20 seconds.

Comparative Example 2

An absorbent body was fabricated in the same manner as Comparative Example 1, except that thermoplastic resin fibers with a fiber length of 12 mm were used instead of thermoplastic resin fibers with a fiber length of 30 mm.

Comparative Example 3

An absorbent body was fabricated in the same manner as Comparative Example 1, except that thermoplastic resin fibers with a fiber length of 6 mm were used instead of thermoplastic resin fibers with a fiber length of 30 mm.

Comparative Example 4

Pulp fibers were opened and accumulated on a suction drum to form a pulp web with a basis weight of 240 $g/m^2$, and then the pulp web was opened and accumulated on an absorbent body-molding drum to obtain a molded article having the shape shown in Fig. 1 (L = 200 mm, W = 70 mm), as an absorbent body.

The absorbent bodies fabricated in the examples and comparative examples were measured for basis weight, thickness, apparent density, tensile strength, displacement at maximum load and Gurley bending resistance. The measurement results are shown in Table 1.

The methods for measuring the properties were as follows.

[Basis weight]

The basis weight was measured according to JIS L 1913:2010 "6.2 Weight per unit area (ISO method)".

More specifically, three 150 mm × 40 mm test pieces were prepared, and the weight of each test piece was measured under standard conditions using an HF-300 electronic scale by Kensei Co., Ltd.

[Thickness]

A FS-60DS thickness gauge by Daiei Kagaku Seiki Mfg. Co., Ltd. (measuring surface: 44 mm diameter, measuring pressure: 3 $g/cm^2$) was used for measurement of the thickness at five different locations of the sample after pressing at 3 $g/cm^2$ for 10 seconds, under standard conditions (temperature: 23±2°C, relative humidity: 50±5%), and the mean value of the measured values at the five locations was recorded as the thickness of the absorbent body.

[Apparent density]

The apparent density was calculated by the following formula.

$$\texttt{Apparent density = basis weight } \div \texttt{ thickness}$$

[Tensile strength]

The tensile strength was measured according to JIS L 1913 6.3.

More specifically, five 150 mm × 25 mm test pieces were prepared and mounted in a tensile tester (AG-1kNI by Shimadzu Corp.) in a steady temperature and humidity room (temperature: 20°C, humidity: 60%), at a grip spacing of 100 mm, a load was applied at a pull rate of 100 mm/min until the test piece broke, and the load (N) at breakage of the test piece, divided by the width of the test piece (2.5 cm) was recorded as the tensile strength (N/cm).

[Displacement at maximum load]

The elongation at maximum load during measurement of the tensile strength was recorded as the displacement at maximum load.

[Gurley bending resistance]

The Gurley bending resistance was measured according to JIS L 1913 6.7.3.
More specifically, five 38 mm × 25 mm test pieces were prepared, and the bending resistance was calculated from the average value for the front and back of each test piece after continuous measurement of the front and back with a Gurley tester (No.311 bending resistance meter by Yasuda Seiki Seisakusho Co., Ltd.).

Table 1

| | Weight ratio of pulp and thermoplastic resin fibers | Fiber lengths of thermoplastic resin fibers (mm) | Treatment | Basis weight of absorbent body (g/m$^2$) | Thickness of absorbent body (mm) | Apparent density of absorbent body (g/cm$^3$) | Tensile strength (N/cm) | Displacement at maximum load (mm) | Gurley bending resistance (mN) |
|---|---|---|---|---|---|---|---|---|---|
| Example 1 | 200:40 | 30 | Embossing | 238 | 2.1 | 0.11 | 0.89 | 22.4 | 0.78 |
| Example 2 | 200:40 | 25 | Embossing | 234 | 2.3 | 0.10 | 0.78 | 20.9 | 0.81 |
| Example 3 | 200:40 | 20 | Embossing | 235 | 2.3 | 0.10 | 0.61 | 20.4 | 0.85 |
| Example 4 | 200:40 | 12 | Embossing | 231 | 2.1 | 0.11 | 0.48 | 10.5 | 0.96 |
| Comp. Ex. 1 | 200:40 | 30 | Hot air | 232 | 2.4 | 0.10 | 3.84 | 35.9 | 2.72 |
| Comp. Ex. 2 | 200:40 | 12 | Hot air | 236 | 2.5 | 0.09 | 3.28 | 34.6 | 1.95 |
| Comp. Ex. 3 | 200:40 | 6 | Hot air | 235 | 2.4 | 0.10 | 3.25 | 23.8 | 1.21 |
| Comp. Ex. 4 | 240:0 | - | None | 241 | 2.5 | 0.10 | 0.35 | 2.39 | 0.72 |

INDUSTRIAL APPLICABILITY

The absorbent body of the invention can be suitably used in a body liquid-absorbing article such as a sanitary napkin.

REFERENCE SIGNS LIST

1 Absorbent body
2 Compressed section
3 Cellulose-based water-absorbent fiber
4 Thermoplastic resin fiber
5 Entangled section
101 Suction drum
102 Recess
103 Material feeder
104 Suction section
105 Mixture of cellulose-based water-absorbent fibers and thermoplastic resin fibers
106 Super-absorbent polymer
107 Super-absorbent polymer feeder
108 Absorbent body
109 Coating machine
110 Carrier sheet
111, 112 Embossing rolls

**Claims**

1. An absorbent body (1) for a body liquid-absorbing article having an apparent density of 0.06 to 0.14 g/cm$^3$ measured as described in the description, comprising 100 parts by weight of cellulose-based water-absorbent fibers (3) and 7.5 to 100 parts by weight of thermoplastic resin fibers (4), wherein
   the thermoplastic resin fibers (4) having longer fiber lengths than the cellulose-based water-absorbent fibers (3) are mutually entangled,
   the absorbent body has compressed sections (2) arranged in a repeating pattern, the compressed sections being non-fused and the absorbent body (1) having a Gurley bending resistance of no greater than 1 mN measured as described in the description,
   the area of each individual compressed section (2) is 0.1 to 20.0 mm$^2$, and
   the proportion of the area of the compressed sections (2) with respect to the area of the absorbent body (1) is 1 to 20%.

2. An absorbent body according to claim 1, wherein the tensile strength of the absorbent body is 0.4 N/cm or greater measured as described in the description.

3. An absorbent body according to claim 1 or 2, wherein the displacement at maximum load of the absorbent body is 10 to 23 mm measured as described in the description.

4. An absorbent body according to any one of claims 1 to 3, wherein the absorbent body further includes a super-absorbent polymer.

5. An absorbent body according to any one of claims 1 to 4, wherein the basis weight of the absorbent body is 40 to 900 g/m$^2$ measured as described in the description.

6. An absorbent body according to any one of claims 1 to 5, wherein at least some of the thermoplastic resin fibers (4) have a first section (4f) that is exposed on one side (6) of the absorbent body, a second section (4s) that is exposed on the other side (7) of the absorbent body, and a connecting section (4c) that connects the first section (4f) and the second section (4s) .

7. A body liquid-absorbing article comprising an absorbent body (1) according to any one of claims 1 to 6.

**Patentansprüche**

1. Saugkörper (1) für einen Körperflüssigkeit absorbierenden Artikel, der eine scheinbare Dichte von 0,06 bis 0,14 g/cm$^3$, gemessen wie in der Beschreibung beschrieben, aufweist, der 100 Gewichtsteile von cellulosebasierten, Wasser absorbierenden Fasern (3) und 7,5 bis 100 Gewichtsteile von thermoplastischen Harzfasern (4) umfasst, wobei

   die thermoplastischen Harzfasern (4), die längere Faserlängen aufweisen als die cellulosebasierten, Wasser absorbierenden Fasern (3), miteinander verwickelt sind,

   der Saugkörper komprimierte Abschnitte (2) aufweist, die in einem wiederholenden Muster angeordnet sind, wobei die komprimierten Abschnitte nicht fusioniert sind und der Saugkörper (1) einen Gurley-Biegewiderstand von nicht größer als 1 mN, gemessen wie in der Beschreibung beschrieben, aufweist,

   die Fläche von jedem einzelnen komprimierten Abschnitt (2) 0,1 bis 20,0 mm$^2$ beträgt und

   der Anteil der Fläche der komprimierten Abschnitte (2) in Bezug auf die Fläche des Saugkörpers (1) 1 bis 20 % beträgt.

2. Saugkörper nach Anspruch 1, wobei die Zugfestigkeit des Saugkörpers 0,4 N/cm oder mehr, gemessen wie in der Beschreibung beschrieben, beträgt.

3. Saugkörper nach Anspruch 1 oder 2, wobei die Verschiebung bei maximaler Belastung des Saugkörpers 10 bis 23 mm, gemessen wie in der Beschreibung beschrieben, beträgt.

4. Saugkörper nach einem der Ansprüche 1 bis 3, wobei der Saugkörper weiter ein superabsorbierendes Polymer einschließt.

5. Saugkörper nach einem der Ansprüche 1 bis 4, wobei das Flächengewicht des Saugkörpers aus 40 bis 900 g/m$^2$, gemessen wie in der Beschreibung beschrieben, beträgt.

6. Saugkörper nach einem der Ansprüche 1 bis 5, wobei zumindest einige der thermoplastischen Harzfasern (4) Folgendes aufweisen: einen ersten Abschnitt (4f), der auf einer Seite (6) des Saugkörpers exponiert ist, einen zweiten Abschnitt (4s), der auf der anderen Seite (7) des Saugkörpers exponiert ist, und einen Verbindungsabschnitt (4c), der den ersten Abschnitt (4f) und den zweiten Abschnitt (4s) verbindet.

7. Körperflüssigkeit absorbierender Artikel, der einen Saugkörper (1) nach einem der Ansprüche 1 bis 6 umfasst.

**Revendications**

1. Garniture absorbante (1) pour un article d'absorption d'un liquide corporel ayant une densité apparente, mesurée comme décrit dans la description, qui va de 0,06 à 0,14 g/cm$^3$ et comprenant 100 parties en poids de fibres à base de cellulose absorbant l'eau (3) et de 7,5 à 100 parties en poids de fibres d'une résine thermoplastique (4), où

   les fibres de résine thermoplastique (4) ayant une longueur plus importante que les fibres à base de cellulose absorbant l'eau (3) sont mutuellement entremêlées,

   la garniture absorbante présente des zones comprimées (2) disposées selon un motif répétitif, les zones comprimées étant non fusionnées et la garniture absorbante (1) ayant une résistance à la flexion, mesurée par la méthode Gurley comme décrit dans la description, qui ne dépasse pas 1 mN,

   l'aire de chaque zone comprimée individuelle (2) va de 0,1 à 20,0 mm$^2$ et

   la proportion de l'aire représentée par les zones comprimées (2), rapportée à l'aire de la garniture absorbante (1), va de 1 à 20 %.

2. Garniture absorbante selon la revendication 1, où la résistance à la traction de la garniture absorbante, mesurée comme décrit dans la description, est égale ou supérieure à 0,4 N/cm.

3. Garniture absorbante selon la revendication 1 ou 2, où le déplacement de la garniture absorbante à une charge maximale, mesuré comme décrit dans la description, va de 10 à 23 mm.

4. Garniture absorbante selon l'une quelconque des revendications 1 à 3, où la garniture absorbante inclut en outre un polymère super-absorbant.

5. Garniture absorbante selon l'une quelconque des revendications 1 à 4, où la masse surfacique de la garniture absorbante, mesurée comme décrit dans la description, va de 40 à 900 g/m$^2$.

6. Garniture absorbante selon l'une quelconque des revendications 1 à 5, où certaines au moins des fibres de résine thermoplastique (4) ont une première zone (4f) qui est exposée d'un côté (6) de la garniture absorbante, une deuxième zone (4s) qui est exposée de l'autre côté (7) de la garniture absorbante et une zone de connexion (4c) qui relie la première zone (4f) et la deuxième zone (4s).

7. Article d'absorption d'un liquide corporel comprenant une garniture absorbante (1) selon l'une quelconque des revendications 1 à 6.

# FIG. 1

EP 3 162 344 B1

FIG. 2

FIG. 3

16

# FIG. 4

**EP 3 162 344 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002011047 A

- US 2003236511 A1